# EUROPEAN PATENT SPECIFICATION

(11) **EP 3 828 263 B1**
(45) Date of publication and mention of the grant of the patent: **15.02.2023**
(21) Application number: 20208393.7
(22) Date of filing: 18.11.2020
(51) Int. Cl.: C12N 5/0775

(54) **METHOD AND KIT FOR PREPARING CLINICAL-GRADE MESENCHYMAL STEM CELLS DERIVED FROM HUMAN INDUCED PLURIPOTENT STEM CELLS**
VERFAHREN UND KIT ZUR HERSTELLUNG VON MESENCHYMALEN STAMMZELLEN VON KLINISCHER QUALITÄT AUS HUMANINDUZIERTEN PLURIPOTENTEN STAMMZELLEN
PROCÉDÉ ET KIT DE PRÉPARATION DE CELLULES SOUCHES MÉSENCHYMATEUSES DE GRADE CLINIQUE DÉRIVÉES DE CELLULES SOUCHES PLURIPOTENTES INDUITES HUMAINES

(30) Priority: 27.11.2019 CN 201911180682
(43) Date of publication of application: 02.06.2021
(73) Proprietor: Shanghai East Hospital (East Hospital Affiliated to Tongji University), Pudong New District Shanghai 200120 (CN)
(72) Inventor: Liu, Zhongmin, Shanghai 200120 (CN); Jia, Wenwen, Shanghai 200120 (CN); Lu, Jizhen, Shanghai 200120 (CN); Tang, Hongming, Shanghai 200120 (CN)
(74) Representative: Canzler & Bergmeier Patentanwälte Partnerschaft mbB

(56) References cited:
- WO-A2-2016/081032
- CARLOS LUZZANI ET AL: "A therapy-grade protocol for differentiation of pluripotent stem cells into mesenchymal stem cells using platelet lysate as supplement", STEM CELL RESEARCH & THERAPY, BIOMED CENTRAL LTD, LONDON, UK, vol. 6, no. 1, 12 January 2015 (2015-01-12), page 6, XP021213512, ISSN: 1757-6512, DOI: 10.1186/SCRT540
- Y. S. CHEN ET AL: "Small Molecule Mesengenic Induction of Human Induced Pluripotent Stem Cells to Generate Mesenchymal Stem/Stromal Cells", STEM CELLS TRANSLATIONAL MEDICINE, vol. 1, no. 2, 1 February 2012 (2012-02-01), pages 83-95, XP055081405, ISSN: 2157-6564, DOI: 10.5966/sctm.2011-0022
- DMITRIY SHEYN ET AL: "Human Induced Pluripotent Stem Cells Differentiate Into Functional Mesenchymal Stem Cells and Repair Bone Defects : iPSC-Derived MSCs Repair Bone Defects", STEM CELLS TRANSLATIONAL MEDICINE, vol. 5, no. 11, 11 July 2016 (2016-07-11), pages 1447-1460, XP055577584, US ISSN: 2157-6564, DOI: 10.5966/sctm.2015-0311
- MAKOTO FUKUTA ET AL: "Derivation of Mesenchymal Stromal Cells from Pluripotent Stem Cells through a Neural Crest Lineage using Small Molecule Compounds with Defined Media", PLOS ONE, vol. 9, no. 12, 2 December 2014 (2014-12-02), pages 1-25, XP055332980, DOI: 10.1371/journal.pone.0112291
- MA YANGYANG ET AL: "Preserving self-renewal of porcine pluripotent stem cells in serum-free 3i culture condition and independent of LIF and b-FGF cytokines", CELL DEATH DISCOVERY, vol. 4, no. 1, 14 February 2018 (2018-02-14), XP055795558, DOI: 10.1038/s41420-017-0015-4 Retrieved from the Internet: URL:http://www.nature.com/articles/s41420- 017-0015-4.pdf>

## Description

### CROSS REFERENCE TO RELATED APPLICATION

This disclosure claims the priority of Chinese Patent Application NO. 201911180682.8 entitled "Method and kit for preparing clinical-grade mesenchymal stem cells derived from human induced pluripotent stem cells" filed with China National Intellectual Property Administration on November 27, 2019,

### TECHNICAL FIELD

The present disclosure belongs to the technical field of cell culture, and particularly relates to a method and a non-claimed kit for preparing clinical-grade mesenchymal stem cells derived from human induced pluripotent stem cells.

### BACKGROUND ART

Mesenchymal stem cells (MSC) are considered a cell population derived from mesoderm and ectoderm in early embryonic development, which have multi-directional differentiation potential and low immunogenicity, and are widely used in regenerative medicine, medical care, cosmetology and other fields. At present, mesenchymal stem cells are mainly derived from umbilical cord, fat, bone marrow and other tissues, with limited passage numbers. There are individual differences in mesenchymal stem cells from different donors, which is difficult to meet the needs for stable and consistent cell sources in clinical applications. In addition, mesenchymal stem cells can differentiate into a variety of terminal cell types when transplanted into the body. During the differentiation process, HLA antigens of cells can be expressed. Therefore, although mesenchymal stem cells themselves are immune tolerant, immune rejection may still occur after mesenchymal stem cells are transplanted for a period of time.

At present, the source of mesenchymal stem cells can be induced by using human pluripotent stem cells (hPSCs). Human pluripotent stem cells usually refer to human embryonic stem cells (hESCs) and human induced pluripotent stem cells (hiPSCs), which have unlimited self-renewal and differentiation into all cell types of individual. At present, there are many ways to induce differentiation from hESCs and hiPSCs into MSCs, for example, the patent with publication number CN 106520687 A discloses a medium containing platelet-derived factor (PDGFAB), dexamethasone and 10% fetal bovine serum (FBS) as a differentiation culture medium for inducing pluripotent stem cells to differentiate into mesenchymal stem cells. The results show that MSC differentiated by hiPSCs cells can self-renew and proliferate well during in vitro culture, and have the ability of osteogenic, adipogenic and chondrogenic differentiation. However, this culture method requires the replacement of two different differentiation media, and the differentiated mesenchymal stem cells can not be used clinically due to the use of FBS.

Reference is made to following documents; the numbering will be adhered to in the rest of the disclosure.

D1: CARLOS LUZZANI ET AL: "A therapy-grade protocol for differentiation of pluripotent stem cells into mesenchymal stem cells using platelet lysate as supplement", STEM CELL RESEARCH & THERAPY, vol. 6, no. 1, 12 January 2015 (2015-01-12), page 6, XP021213512.

D2: Y. S. CHEN ET AL: "Small Molecule Mesengenic Induction of Human Induced Pluripotent Stem Cells to Generate Mesenchymal Stem/Stromal Cells", STEM CELLS TRANSLATIONAL MEDICINE, vol. 1, no. 2, 1 February 2012 (2012-02-01), pages 83-95, XP055081405.

D3: DMITRIY SHEYN ET AL: "Human Induced Pluripotent Stem Cells Differentiate Into Functional Mesenchymal Stem Cells and Repair Bone Defects: iPSC-Derived MSCs Repair Bone Defects", STEM CELLS TRANSL ATIONAL MEDICINE, vol. 5, no. 11, 11 July 2016 (201 6-07-1 1), pages 1447-1460, XP055577584.

D4: MAKOTO FUKUTA ET AL: "Derivation of Mesenchymal Stromal Cells from Pluripotent Stem Cells through a Neural Crest Lineage using Small Molecule Compounds with Defined Media", PLOS ONE, vol.9, no. 12, 2 December 2014 (2014-12-02), pages 1-25, XP055332980.

D5: WO 2016/081032 A2 (TEXAS A & M UNIV SYS [US]; TEMPLE THERAPEUTICS INC [US] ET AL.) 26 May 2016 (2016-05-26).

D6 MA YANGYANG ET AL: "Preserving sellf-renewal of porcine pluripotent stem cells in serum-free 3i culture condition and independent of LIF and b-FGF cytokines", CELL DEATH DISCOVERY, vol. 4, no. 1, 14 February 2018 (2018-02-14), XP055795558.

Document D1 discloses a therapy-grade protocol for differentiation of pluripotent stem cells (hESC or iPSC) into mesenchymal stem cells using platelet lysate (10%) as supplement in alpha modified Eagle's medium (alpha MEM) for growth on Matrigel-/ Geltrex-coated plates, includes a medium contained further a Rho kinase inhibitor (Y27632) and B27 (1:100).

D2 describes generation of mesenchymal stem cells from induced pluripotent stem cells in presence of TGFbeta pathway inhibitor SB431542. A treatment with SB431542 in two-dimensional cultures followed by culture-induced epithelial-to-mesenchymal transition leads to rapid and uniform MSC conversion of human pluripotent cells without a need for embryoid body formation or feeder cell co-culture, providing a robust, clinically applicable, and efficient system for generating MSCs from human iPSCs. No alpha-MEM, CHIR99021 and platelet extractlysate is mentioned in D2.

Prior art D3 discloses a differentiation of human iPSC into mesenchymal stem cells in presence of a glycogensynthase kinase 3beta inhibitor of a Wnt/beta-catenin signaling pathway (CHIR99021) in IMDM basal medium. D3 does therefore not discloses alpha-MEM, SB431 542 and platelet extractlysate.

D4 discloses derivation of mesenchymal stem cells from hiPSC in presence of SB431542 and CHIR99021 in DMEM on ECM coated plates. D4 does neither discloses alpha-MEM nor platelet extract/lysate.

Patent application D5 describes a method of producing mesenchymal stem cells from induced pluripotent stem cells, comprising: (a) culturing said induced pluripotent stem cells in a medium (DMEM) containing a TGF-beta inhibitor (SB431 542) and in an atmosphere containing from about 7 vol. % to about 8 vol. % CO₂ for a period of time of from about 20 days to about 35 days; and (b) transferring said cells from step (a) to a culture dish having a hydrophilic surface and culturing said cells in a medium containing a TGF-beta inhibitor for a period of time-sufficient to produce mesenchymal stem cells.

Document D6 discloses a medium (DMEM) supplemented with 3 micromolar CHIR99021, 2 micromolar SB431542 and 7% platelet lysate for preserving self-renewal of porcine pluripotent stem cells.

None of D1-D6 discloses a medium and associated matter as required by the independent claims, i.e. a medium based on alpha-MEM comprising human platelet extract/lysate, SB431542 and CHIR99021, let alone in the indicated concentration ranges.

### SUMMARY OF THE INVENTION

In view of this, the purpose of the disclosure is to provide a non-claimed induction medium for the differentiation of human induced pluripotent stem cells into mesenchymal stem cells, and the mesenchymal stem cells induced by the induction medium is able to be used clinically.

The present disclosure also provides a non-claimed kit for the differentiation of human induced pluripotent stem cells into mesenchymal stem cells, which is used in the process of producing clinical-grade mesenchymal stem cells.

The present disclosure also provides a method for preparing clinical-grade mesenchymal stem cells derived from human induced pluripotent stem cells.

The present disclosure also provides a non-claimed induction medium for the differentiation of human induced pluripotent stem cells into clinical-grade mesenchymal stem cells, which takes an α-MEM medium as a basic medium, and also includes the following components: 5%-10% human platelet extract in volume concentration, 5-10 µmol/L SB431542 and 1-5µmol/L CHIR99021.

In some embodiments, the non-claimed induction medium includes the following components: 10% human platelet extract in volume concentration, 10 µmol/L SB431542 and 2 µmol/L CHIR99021.

The present disclosure also provides a non-claimed kit for the differentiation of human induced pluripotent stem cells into clinical-grade mesenchymal stem cells, which includes the induction medium.

In some embodiments, the non-claimed kit also includes an amplification medium for amplification culture of mesenchymal stem cells.

The mesenchymal stem cells amplification medium takes an α-MEM medium as a basic medium and also includes 5%-10% human platelet extract in volume concentration.

The present disclosure provides a non-claimed use of the induction medium or the kit in the differentiation of human induced pluripotent stem cells into clinical-grade mesenchymal stem cells.

The present disclosure provides a method for preparing clinical-grade mesenchymal stem cells derived from human induced pluripotent stem cells, which includes the steps of:
1) digesting human induced pluripotent stem cells to prepare single cell suspension, and plating it on a pretreated culture plate;
2) induction culturing by using the induction medium when the cells reaching 80% confluence, and changing the medium every day;
3) digesting the cells to prepare single cell suspension when the cells reaching 100% confluence, inoculating on an amplification medium for amplification culture, and the cells obtained after 3-4 generations of amplification culture are clinical-grade mesenchymal stem cells.

In some embodiments, the reagent for digestion in step 1) or step 3) is TrypLE select.

In some embodiments, the pretreatment method for the pretreated culture plate in step 1) is adding a coating buffer to the culture plate for incubation coating to obtain the pretreated culture plate;
the coating buffer takes a DPBS solution containing calcium and magnesium ions as basic solution, and also includes Laminin 521, Laminin511, Geltrax or Vitronectin; the concentration of Laminin 521, Laminin511, Geltrax or Vitronectin is 5 µg/mL;
the method of the incubation coating includes incubating in a refrigerator at 2-8 ^{°}C overnight or incubating at 37 ^{°}C for 1-2h. In some embodiments, the cell density at the time of inoculation in step 1) or step 3) is 1-5 × 10⁴/cm². In some embodiments, the temperature of induction culture in step 2) or amplification culture in step 3) is 37 ^{°}C, and the volume concentration of CO₂ during the induction culture in step 2) or the amplification culture in step 3) is 5%.

The present disclosure provides a method for preparing clinical-grade mesenchymal stem cells derived from human induced pluripotent stem cells, using GMP-grade reagents as differentiation induction medium components, and using clinical-grade cell culture methods, which can efficiently directionally differentiate clinical-grade human induced pluripotent stem cells into mesenchymal stem cells, and the expression of positive characteristic markers is up to 90% or more, while the negative markers are not expressed; the mesenchymal stem cells can be differentiated into bone, cartilage, fat and other cell types in vitro. The mesenchymal stem cells prepared by the method provided by the present disclosure are mesenchymal stem cells with uniform source, stable supply and consistent characteristics for clinical applications.

### BRIEF DESCRIPTION OF THE DRAWINGS

FIG. 1 shows the cell morphology of different differentiation methods at different times, and each scale in the figure is 100 µm;
FIG. 2 shows the comparative results of the expression of specific surface markers of P4 generation mesenchymal stem cells with different differentiation methods, in which FIG. 2-1 shows the expression of CD105, CD73 and CD90, and FIG. 2-2 shows the expression of CD11, CD19 and CD34. FIG. 2-3 shows the expression of CD45 and HLA-DR;
FIG. 3 shows the comparative results of osteogenic, chondrogenic and adipogenic differentiation capabilities of P5 generation mesenchymal stem cells cultured by different differentiation methods; each scale in the figure is 100 µm;
FIG. 4 shows the results of mRNA expression levels of anti-inflammatory cytokines IL-10, PGE2, IDO, pro-inflammatory cytokines IL-6 and TGF-β of different generations of cells prepared by the differentiation method provided by the present disclosure.

### DETAILED DESCRIPTION OF THE EMBODIMENTS

The present disclosure provides an induction medium for the differentiation of human induced pluripotent stem cells into clinical-grade mesenchymal stem cells, which takes an α-MEM culture medium as a basic medium, and also includes the following components: 5%-10% human platelet extract in volume concentration, 5-10 µmol/L SB431542 and 1-5µmol/L CHIR99021.

The induction medium provided by the present disclosure takes α-MEM medium as a basic medium. There is no particular limitation on the formula and preparation method of the α-MEM medium, and the formula and preparation method of the α-MEM medium well known in the art will do. In an embodiment of the present disclosure, the α-MEM medium was purchased from Thermo Fisher Scientific.

The induction medium provided by the present disclosure includes human platelet extract. The volume concentration of the human platelet extract is preferably 10%. The human platelet extract belongs to GMP-grade serum substitute, and its functions are as follows: it is rich in growth factors and cytokines required for cell growth and proliferation, and can replace fetal bovine serum (FBS) for culturing adipose-derived (AD), Bone marrow (BM) or umbilical cord (UC) mesenchymal stem cells (MSCs). The human platelet extract provides the basic growth environment for MSC in the induction mediums. There is no particular limitation on the source of the human platelet extract, the product well known in the art will do. In an embodiment of the present disclosure, the human platelet extract was purchased from UltraGRO^{™}-Advanced, Cat# HPCFDCGL50, Helios Bioscience.

The induction medium provided by the present disclosure includes SB431542. The concentration of SB431542 is preferably 10 µmol/L. The function of SB431542 is to induce human pluripotent stem cells to differentiate into mesectoderm as an inhibitor of TGF-0, ALK4 and ALK7. There is no particular limitation on the source of the SB431542, the source of SB431542 well known in the art will do. The SB431542 was purchased from Tocris Bioscience, Catalog number: TB1614-GMP.

The induction medium provided by the present disclosure includes CHIR99021. The concentration of CHIR99021 is preferably 2 µmol/L. The CHIR99021 is used as an inhibitor of GSK-3β and can activate Wnt3a signal, thereby inducing human pluripotent stem cells to differentiate into mesentoderm. The combination of CHIR99021 and SB431542 can induce human pluripotent stem cells to differentiate into mesoderm cells and further form mesenchymal stem cells under appropriate concentration and action time, which has obvious advantages over other combination schemes in inducing effects. In an embodiment of the present disclosure, the CHIR99021 is purchased from Tocris Bioscience, and the Catalog number is TB4423-GMP.

In further possible embodiments other than the ones claimed by the claims, there is no particular limitation on the preparation method of the induction medium, the preparation method of the induction medium well known in the art will do.

The present disclosure provides a kit for the differentiation of human induced pluripotent stem cells into clinical-grade mesenchymal stem cells, which includes the induction medium. The kit also preferably includes an amplification medium for amplification culture of mesenchymal stem cells; the mesenchymal stem cells amplification medium takes an α-MEM medium as a basic medium and also includes 5%-10% human platelet extract in volume concentration, more preferably includes 10% human platelet extract. The induction medium and the amplification medium are packaged separately. There is no particular limitation on the volume of induction medium and amplification medium in the kit according to the disclosure.

The present disclosure provides a use of the induction medium or the kit in the differentiation of human induced pluripotent stem cells into clinical-grade mesenchymal stem cells.

The present disclosure provides a method for preparing clinical-grade mesenchymal stem cells derived from human induced pluripotent stem cells, which includes the steps of:
1) digesting human induced pluripotent stem cells to prepare single cell suspension, and plating it on a pretreated culture plate;
2) induction culturing by using the induction medium when the cells reaching 80% confluence, and changing the medium every day;
3) digesting the cells to prepare a single cell suspension when the cells reaching 100% confluence, inoculating on an amplification medium for amplification culture, and the cells obtained after 3-4 generations of amplification culture are clinical-grade mesenchymal stem cells.

In some embodiments, human induced pluripotent stem cells were digested into single cell suspension, and the single cell suspension was inoculated on the pretreated culture plate.

In some embodiments, the method for preparing human induced pluripotent stem cells is to separate and extract umbilical cord blood mononuclear cells, and transfer Yamanaka transcription factors into umbilical cord blood mononuclear cells by using Sendai virus or liposome carriers to obtain induced pluripotent stem cells. The obtained human induced pluripotent stem cells can renew themselves indefinitely when cultured in vitro, and can differentiate into endoderm, mesoderm, and ectoderm-specific cell types after induction. The obtained human induced pluripotent stem cells can be injected into nude mice to form teratomas with the characteristics of three germ layers.

In some embodiments, the reagent for digestion preferably is TrypLE select. The cell density at the time of inoculation is preferably 1-5×10⁴/cm², more preferably 2-3×10⁴/cm².

In some embodiments, the pretreatment method for the pretreated culture plate is preferably to add a coating buffer to the culture plate for incubation coating to obtain the pretreated culture plate; the coating buffer takes a DPBS solution containing calcium and magnesium ions as basic solution, and also includes Laminin 521, Laminin 511, Geltrax or Vitronectin; the concentration of Laminin 521, Laminin 511, Geltrax or Vitronectin is 5 µg/mL. The Laminin 521, Laminin 511, Geltrax or Vitronectin treatment of the culture plate is beneficial to the adherent growth of human induced pluripotent stem cells. The incubation coating preferably includes two incubation methods, one is to incubate in a refrigerator at 2-8 ^{°}C overnight, and the other is to incubate at 37 ^{°}C for 1-2h. The coating buffer is discarded after the incubation coating. The basic solution was purchased from Invitrogen Gibco.

The induction medium is used for induction culture when the cells reach 80% confluence, and the medium is changed every day.

In some embodiments, the temperature of induction culture is preferably 37 ^{°}C. The volume concentration of CO₂ during the induction culture is preferably 5%. It usually takes 3-4 days for the cells to reach 80% confluence.

The cells are digested to prepare single cell suspension when the cells reach 100% confluence, the single cell suspension is inoculated on an amplification medium for amplification culture, and the cells obtained after 3-4 generations of amplification culture are clinical-grade mesenchymal stem cells.

In some embodiments, the reagent for digestion preferably is TrypLE select. The cell density at the time of inoculation for amplification culture is preferably 1-5×10⁴/cm², more preferably 5×10⁴/cm². The temperature of amplification culture is preferably 37 ^{°}C. The volume concentration of CO₂ during the amplification culture is preferably 5%. The inoculation density is preferably 1-2×10⁴/cm² in the process of 3-4 generations amplification culture.

In some embodiments, the expressions of 8 specific surface markers (including 3 positive markers and 5 negative markers) on the prepared clinical-grade mesenchymal stem cells are tested, and it is found that none of the negative markers of MSC are expressed, the expression proportions of positive markers CD105 and CD73 are both larger than 95%. As for CD90, the induction medium provided by the present disclosure is used for differentiation, and the expression proportion of CD90 expressed by mesenchymal stem cells reaches 89.4%, which is significantly higher than the other two control groups (the expression proportion of makers is the ratio of the number of fluorescently labeled cells to the total number). At the same time, the experimental results of clinical-grade mesenchymal stem cells in adipogenic, osteogenic, and chondrogenic differentiation show that the mesenchymal stem cells obtained by the method provided by the present disclosure have better differentiation capabilities such as adipogenic, osteogenic, and cartilage.

The method and non-claimed kit for preparing clinical-grade mesenchymal stem cells derived from human induced pluripotent stem cells provided by the present disclosure will be described in detail below with reference to examples, but they should not be understood as any limitation to the scope of the present disclosure.

### EXAMPLE 1

Formula of MSC induction medium (M+S+C2 for short):
1L α-MEM basic medium contained 10% human platelet extract, 10µmol/L SB431542 and 2µmol/L CHIR99021. The MSC induction medium was obtained after conventional sterilization.

### EXAMPLE 2

Formula of MSC induction medium:
1L α-MEM basic medium contained 10% human platelet extract, 5µmol/L SB431542 and 5µmol/L CHIR99021. The MSC induction medium was obtained after conventional sterilization.

### EXAMPLE 3

Formula of MSC induction medium:
1L α-MEM basic medium contained 10% human platelet extract, 10µmol/L SB431542 and 2µmol/L CHIR99021. The MSC induction medium was obtained after conventional sterilization.

### EXAMPLE 4

Method for preparing clinical-grade mesenchymal stem cells derived from human induced pluripotent stem cells
1) Human iPS cells were digested into single cells with TrypLE select, and the single cells were inoculated into a culture plate coated with Laminin 521 (optional Laminin 511, Geltrax, Vitronectin, etc.) at 1×10⁴/cm²; herein the laminin 521 coating: Laminin 521 was diluted with DPBS containing calcium and magnesium ions one day in advance to a final concentration of 5µg/mL, the diluted solution was added to the culture plate, and incubated in a refrigerator at 2-8 ^{°}C overnight for use. Before use, the Laminin coating buffer was discarded.
2) After the cells were cultured for 4 days, the MSC induction medium prepared in Examples 1 to 3 was replaced when the cells reached 80% confluence, and the medium was changed every day.
3) After the cells were induction cultured for 10 days, the cells were digested into single cells with TryPLE express when the cells reached 100% confluence, and the single cells were inoculated into a culture dish according to the cell density of 5×10⁴/cm², cultured in MSC amplification medium, which is designated as P0.
4) The cells were digested into single cells with TryPLE express when the cells reached 80% confluence, and the single cells were inoculated into a culture dish according to the cell density of 2×10⁴/cm², cultured in MSC amplification medium, which is designated as P1.
5) The cells were digested into single cells with TryPLE express when the cells reached 80% confluence, and the single cells were inoculated into a culture dish according to the cell density of 1×10⁴/cm², cultured in MSC amplification medium, which is designated as P2.
6) After that, the cells were inoculated according to the cell density of 1×10⁴/cm² for each passage.
7) Cells≥P3 generation could be used for subsequent experiments or cryopreservation.

### COMPARATIVE EXAMPLE 1

Formula of MSC induction medium (M for short):
1L α-MEM basic medium contained 10% human platelet extract. The MSC induction medium was obtained after filtration and sterilization. Mesenchymal stem cells were induced according to the method of Example 4.

### COMPARATIVE EXAMPLE 2

Formula of MSC induction medium (M+B+S+C2 for short):
1L α-MEM basic medium contained 10% human platelet extract, 2% B27, 10µmol/L SB431542 and 2µmol/L CHIR99021. The MSC induction medium was obtained after filtration and sterilization. Mesenchymal stem cells were induced according to the method of Example 4.

### EXAMPLE 5

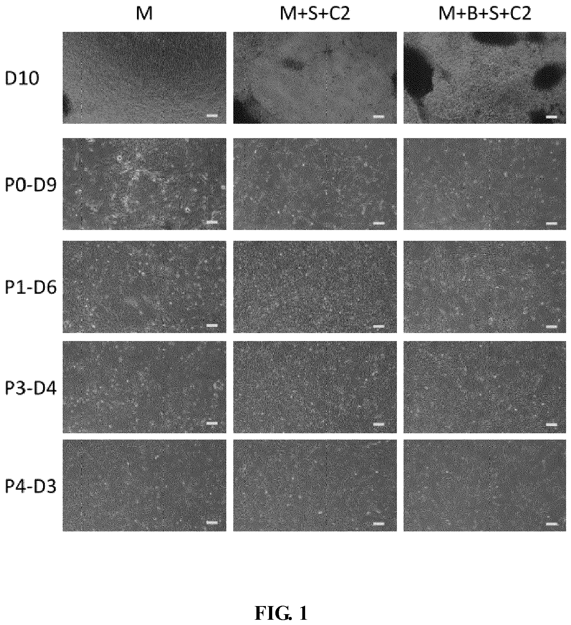

The mesenchymal stem cells induced in Example 4, Comparative Example 1 and Comparative Example 2 were observed for cell morphology under an optical microscope. The results are shown in FIG. 1, where D10 is a photo taken after iPS was cultured with induction medium for 10 days; P0-D9 are photos taken when P0 generation cells were cultured for 9 days, P1-D6 are photos taken when P1 generation cells were cultured for 6 days, and P3-D4 are photos taken when P3 generation cells were cultured for 4 days, P4-D3 are photos taken when P4 generation cells were cultured for 3 days.

It can be seen from FIG. 1 that the cells induction cultured in the induction medium (M+S+C2) provided by the present disclosure had a more uniform cell morphology (P0-D9) in the P0 generation, and the cells proliferated faster (P1-D6, P3-D4) when passaged at the same density; the cell bodies induced by the induction medium used in Comparative Example 2 (M+B+S+C2 group) were significantly larger than the first two groups (M+S+C2 group and M group); as the larger the cell body, the slower the proliferation, i.e. the larger the cell body is not conducive to cell proliferation. In the prior art, B27 was added to the α-MEM+ platelet extract medium for inducing the differentiation of mesenchymal stem cells, but the results of this example showed that the addition of B27 was not conducive to the proliferation and differentiation of mesenchymal stem cells.

### EXAMPLE 6

The mesenchymal stem cells induced in Example 4, Comparative Example 1 and Comparative Example 2 were tested for the expression of 8 specific surface markers, and the specific positive markers were CD105, CD73, CD90; negative markers were CD11, CD19, CD34, CD45, HLA-DR. Direct-labeled fluorescent antibodies in the flow cytometric of 8 markers (CD105, CD73, CD90, CD11, CD19, CD34, CD45, and HLA-DR) were all purchased from R&D system. Specifically, MSC cells of P4 generation were digested into single cells, which were mixed with the above 8 marker antibodies, the mixture was incubated in the dark at room temperature for 30 minutes, the solution incubated was washed twice with phosphate buffer, and the expression ratio of markers was detected by flow cytometry.

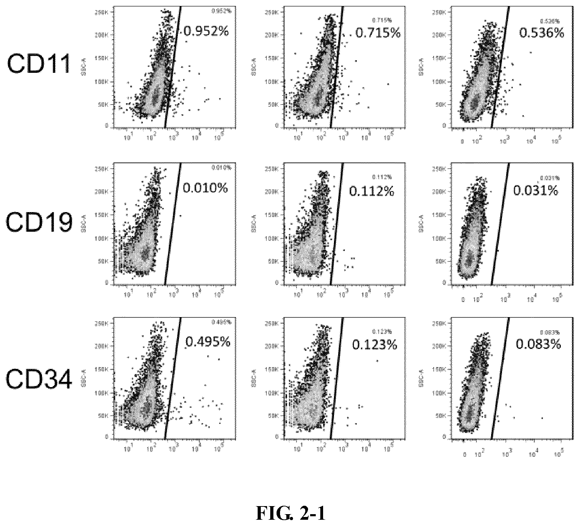

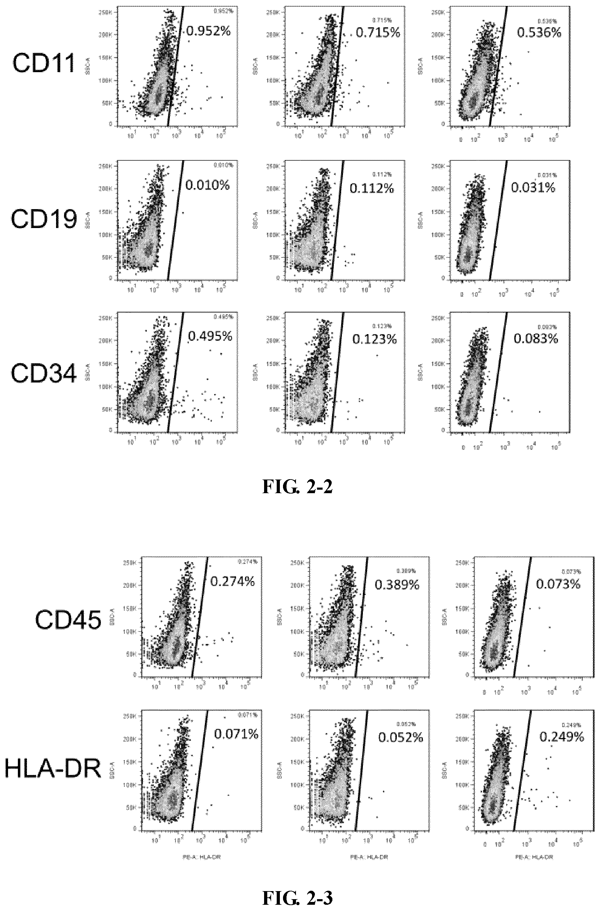

The results are shown in FIG. 2. FIG. 2 shows the comparison of the expression of specific surface markers of P4 generation mesenchymal stem cells with different differentiation methods.

The results showed that none of the negative markers were expressed on the MSCs differentiated from the three induction media, and the expression proportions of positive markers CD 105 and CD73 are both larger than 95%. As for CD90, the expression proportion of CD90 on MSCs induced by the scheme M+S+C2 reached 89.4%, which was significantly higher than the other two groups (M: 50.8%; M+B+S+C2: 35.1%). Therefore, the induction medium of M+S+C2 is more effective in inducing the differentiation of pluripotent stem cells into mesenchymal stem cells.

### EXAMPLE 7

The iPS-MSCs was cultured in M medium, M+S+C2 medium and M+B+S+C2 respectively. The iPS-MSCs of P5 generation were chondrogenically differentiated according to the instructions of StemPro^{™} Chondrogenesis Differentiation Kit(Gibco, A1007101), and the cartilages were stained with Alcian blue staining. The iPS-MSCs were adipogenically differentiated according to the instructions of StemPro^{™} Adipogenesis Differentiation Kit (A1007001), and the adipocytes were stained with Oil Red O staining. The iPS-MSCs were osteogenically differentiated according to the instructions of StemPro^{™} Osteogenesis Differentiation Kit(Gibco, A1007201), and the osteoblasts were stained with Alizarin Red Staining Solution.

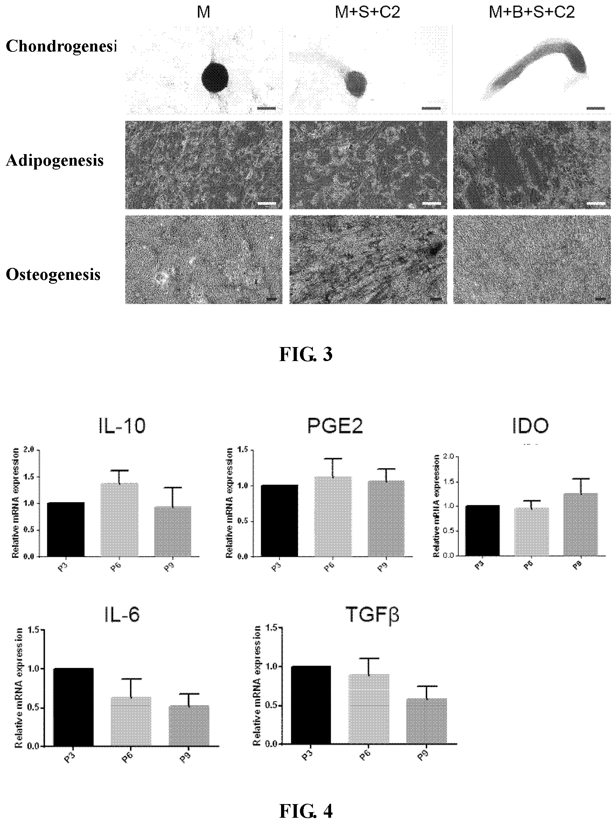

The results are shown in FIG. 3. It can be seen from FIG. 3 that there is no significant difference in chondrogenic and adipogenic differentiation ability among the three groups of cells, while the osteogenic differentiation ability of the cells in M+B+S+C2 group is significantly better than that of the cells in other groups

### EXAMPLE 8

The cells of different generations (P3, P6, P9) cultured in M+S+C2 medium were collected, and the total RNA was extracted by using RNA simple kit (Tiangen, DP491) for total RNA extraction, the cells were reversed transcription by using FastKing cDNA first strand synthesis kit (Tiangen, KR118), and the cells were analyzed through qRT-PCR by using GoTaq^{®} qPCR and RT-qPCR Systems (Promega, A6001) . The reaction system was as follows: 10 µL of Go Taq qPCR Master Mix (2×) , 0.2 µL of 10 µM forward primer, 0.2 µL of 10 µM reverse primer, 1 µL of cDNA template, and 8.6 µL of ddH₂O, the total amount was 20 µL; the reaction program was as follows: pre-denaturing: 95 ^{°}C, 10min; 95 ^{°}C, 20s; 60 ^{°}C, 30s; 72 ^{°}C, 30s, 40 cycles. The changes in the mRNA expression levels of anti-inflammatory cytokines IL-10, PGE2 and IDO, and pro-inflammatory cytokines IL-6 and TGFβ were detected. The relevant primer sequences are shown in Table 1.

**Table 1 Nucleotide sequence information for primers**

| | |
|---|---|
| hTGFβ_Forward | GGCCAGATCCTGTCCAAGC (SEQ ID NO.1) |
| hTGFβ_Revers | GTGGGTTTCCACCATTAGCAC (SEQ ID NO.2) |
| hIL-6_Forward | CCTGAACCTTCCAAAGATGGC < SEQ ID NO.3) |
| hIL-6_Revers | TTCACCAGGCAAGTCTCCTCA < SEQ ID NO.4) |
| hIDO1_Forward | GCCAGCTTCGAGAAAGAGTTG (SEQ ID NO.5) |
| hIDO1_Revers | ATCCCAGAACTAGACGTGCAA (SEQ ID NO.6) |
| hPGE2_Forward | CGATGCTCATGCTCTTCGC < SEQ ID NO.7) |
| hPGE2_Revers | GGGAGACTGCATAGATGACAGG (SEQ ID NO.8) |
| hIL-10_Forward | GACTTTAAGGGTTACCTGGGTTG < SEQ ID NO.9) |
| hIL-10_Revers | TCACATGCGCCTTGATGTCTG < SEQ ID NO.10) |

The results were shown in FIG. 4. The expression levels of anti-inflammatory factors such as IL-10, PGE2 and IDO were almost unchanged with the increase of passage numbers (p>0.05). However, the expression levels of pro-inflammatory cytokines IL-6 and TGFβ were gradually decreased with the increase of passage numbers (^{∗}P≤0.05).

## Claims

1. A method for preparing clinical-grade mesenchymal stem cells derived from human induced pluripotent stem cells, comprising the steps of:
1) digesting human induced pluripotent stem cells to prepare single cell suspension, and plating it on a pretreated culture plate;
2) induction culturing by using an induction medium for the differentiation of human induced pluripotent stem cells into clinical-grade mesenchymal stem cells when the cells obtained in step 1) reaching 80% confluence, and changing the medium every day;
the induction medium takes an α-MEM medium as a basic medium, and adds the following components: 5%-10% human platelet extract in volume concentration, 5-10 µmol/L SB431542 and 1-5µmol/L CHIR99021;
3) digesting the cells to prepare single cell suspension when the cells obtained in step 2) reaching 100% confluence, inoculating on a mesenchymal stem cells amplification medium for amplification culture, and the cells obtained after 3-4 generations of amplification culture are clinical-grade mesenchymal stem cells;
the amplification medium takes an α-MEM medium as a basic medium and adds 5%-10% human platelet extract in volume concentration;
the cell density at the time of inoculation in step 1) or step 3) is 1×10⁴-5×10⁴/cm².

2. The method according to claim 1, wherein the reagent for digestion in step 1) or step 3) is TrypLE select.

3. The method according to claim 1, wherein the pretreatment method for the pretreated culture plate in step 1) is adding a coating buffer to the culture plate for incubation coating to obtain the pretreated culture plate;
the coating buffer takes a DPBS solution containing calcium and magnesium ions as basic solution, and further comprises Laminin 521, Laminin511 or Vitronectin; the concentration of Laminin 521, Laminin511 or Vitronectin is 5 µg/mL;
the method of the incubation coating comprising incubating in a refrigerator at 2-8 ^{°}C overnight or incubating at 37 ^{°}C for 1-2h.

4. The method according to claim 1, wherein the temperature of induction culture in step 2) or amplification culture in step 3) is 37 ^{°}C, and the volume concentration of CO₂ during the induction culture in step 2) or the amplification culture in step 3) is 5%.

5. The method according to claim 1, wherein the concentration of components added in the induction medium is as follows: 10% human platelet extract in volume concentration, 10 µmol/L SB431542 and 2 µmol/L CHIR99021.

## Patentansprüche

1. Verfahren zum Herstellen von mesenchymalen Stammzellen klinischer Qualität, die von menschlichen induzierten pluripotenten Stammzellen abgeleitet sind, umfassend die folgenden Schritte:
1) Digerieren menschlicher induzierter pluripotenter Stammzellen zur Herstellung einer Einzelzellsuspension und Ausplattierung dieser auf einer vorbehandelten Kulturplatte;
2) Induktionskultivierung unter Verwendung eines Induktionsmediums zur Differenzierung menschlicher induzierter pluripotenter Stammzellen in mesenchymale Stammzellen klinischer Qualität, wenn die in Schritt 1) gewonnenen Zellen 80 % Konfluenz erreichen, und täglicher Wechsel des Mediums;
das Induktionsmedium verwendet ein α-MEM-Medium als ein Basismedium , dem die folgenden Komponenten hinzugefügt werden: 5 %-10 % menschlicher Thrombozytenextrakt in Volumenkonzentration, 5-10 µmol/l SB431542 und 1-5 µmol/l CHIR99021;
3) Digerieren der Zellen zur Herstellung einer Einzelzellsuspension, wenn die in Schritt 2) erhaltenen Zellen 100 % Konfluenz erreichen, Inokulation auf ein Amplifikationsmedium für mesenchymale Stammzellen zur Amplifikationskultur, und die nach 3-4 Generationen der Amplifikationskultur erhaltenen Zellen sind mesenchymale Stammzellen klinischer Qualität;
das Amplifikationsmedium verwendet ein α-MEM-Medium als ein Basismedium , dem 5-10 % menschlicher Thrombozytenextrakt in Volumenkonzentration hinzugefügt werden;
die Zelldichte zur Zeit der Inokulation in Schritt 1) oder Schritt 3) beträgt 1×10⁴-5×10⁴/cm².

2. Verfahren nach Anspruch 1, wobei das Reagens für das Digerieren in Schritt 1) oder Schritt 3) TrypLE Select ist.

3. Verfahren nach Anspruch 1, wobei das Vorbehandlungsverfahren für die vorbehandelte Kulturplatte in Schritt 1) das Hinzufügen eines Beschichtungspuffers zu der Kulturplatte zur Inkubationsbeschichtung ist, um die vorbehandelte Kulturplatte zu erhalten;
der Beschichtungspuffer verwendet eine DPBS-Lösung, die Calcium- und Magnesiumionen enthält, als Basislösung, und umfasst ferner Laminin 521, Laminin 511 oder Vitronectin; die Konzentration von Laminin 521, Laminin 511 oder Vitronectin beträgt 5 µg/ml;
wobei das Verfahren der Inkubationsbeschichtung das Inkubieren in einem Kühlschrank bei 2-8 °C über Nacht oder das Inkubieren bei 37 °C für 1-2 h umfasst.

4. Verfahren nach Anspruch 1, wobei die Temperatur der Induktionskultur in Schritt 2) oder der Amplifikationskultur in Schritt 3) 37 °C beträgt und die Volumenkonzentration von CO₂ während der Induktionskultur in Schritt 2) oder während der Amplifikationskultur in Schritt 3) 5 % beträgt.

5. Verfahren nach Anspruch 1, wobei die Konzentration der dem Induktionsmedium hinzugefügten Komponenten wie folgt ist: 10 % menschlicher Thrombozytenextrakt in Volumenkonzentration, 10 µmοl/l SB431542 und 2 µmol/l CHIR99021.

## Revendications

1. Procédé pour la production de cellules souches mésenchymateuses de qualité clinique dérivées de cellules souches pluripotentes induites humaines, comprenant les étapes de :
1) digestion de cellules souches pluripotentes induites humaines pour préparer une suspension de cellules individuelles, et étalement de celle-ci sur une plaque de culture prétraitée ;
2) induction de la culture au moyen d'un milieu d'induction pour la différenciation de cellules souches pluripotentes induites humaines en cellules souches mésenchymateuses de qualité clinique lorsque les cellules obtenues dans l'étape 1) atteignent une confluence de 80 %, et changement du milieu chaque jour ;
le milieu d'induction comporte en tant que milieu de base un milieu α-MEM, et avec addition des composants suivants : 5 %-10 % en concentration en volume d'extrait de plaquettes humaines, 5-10 µmoles/l de SB431542 et 1-5 µmoles/l de CHIR99021 ;
3) digestion des cellules pour obtenir une suspension de cellules individuelles lorsque les cellules obtenues dans l'étape 2) atteignent une confluence de 100 %, inoculation sur un milieu d'amplification de cellules souches mésenchymateuses pour culture d'amplification, et les cellules obtenues après 3-4 générations de culture d'amplification sont des cellules souches mésenchymateuses de qualité clinique ;
le milieu d'amplification comporte en tant que milieu de base un milieu α-MEM et avec addition de 5 %-10 % en concentration en volume d'extrait de plaquettes humaines ;
la densité de cellules au moment de l'inoculation dans l'étape 1) ou l'étape 3) vaut 1×10⁴-5×10⁴/cm².

2. Procédé selon la revendication 1, dans lequel le réactif pour la digestion dans l'étape 1) ou l'étape 3) est TrypLE Select.

3. Procédé selon la revendication 1, dans lequel le procédé de prétraitement pour la plaque de culture prétraitée dans l'étape 1) consiste à ajouter un tampon de tapissage à la plaque de culture pour tapissage d'incubation afin d'obtenir la plaque de culture prétraitée ;
le tampon de tapissage comporte en tant que solution de base une solution DPBS contenant des ions calcium et magnésium, et comprend en outre de la laminine 521, de la laminine 511 ou de la vitronectine ; la concentration de laminine 521, laminine 511 ou vitronectine est de 5 µg/ml ;
le procédé du tapissage d'incubation comprenant une incubation dans un réfrigérateur à 2-8 °C pendant une nuit ou une incubation à 37 °C pendant 1-2 h.

4. Procédé selon la revendication 1, dans lequel la température de la culture d'induction dans l'étape 2) ou de la culture d'amplification dans l'étape 3) est de 37 °C, et la concentration en volume de CO₂ pendant la culture d'induction dans l'étape 2) ou la culture d'amplification dans l'étape 3) est de 5 %.

5. Procédé selon la revendication 1, dans lequel la concentration des composants ajoutés au milieu d'induction est comme suit : 10 % d'extrait de plaquettes humaines en concentration en volume, 10 µmoles/l de SB431542 et 2 µmoles/l de CHIR99021.
